# EUROPEAN PATENT APPLICATION

(11) **EP 2 669 296 A1**
(43) Date of publication of application: **04.12.2013**
(21) Application number: 12739012.8
(22) Date of filing: 17.01.2012
(51) Int. Cl.: C07K 16/24, G01N 33/53, G01N 33/543, G01N 33/576, G01N 33/577

(54) **ANTI-IL28B ANTIBODY AND METHOD FOR ASSAYING IL28B USING SAME**

(30) Priority: 27.01.2011 JP 2011015176
(71) Applicant: FUJIREBIO INC., Shinjuku-ku Tokyo 163-0410 (JP)
(72) Inventor: FUJII, Nobuyuki, Tokyo 163-0410 (JP); SHIRAKAWA, Takashi, Tokyo 163-0410 (JP); OMI, Kazuya, Tokyo 163-0410 (JP)
(74) Representative: Keirstead, Tanis Evelyne
(86) International application number: PCT/JP2012/050794
(87) International publication number: WO 2012/102126

(57) **Abstract**

Disclosed is a novel antibody which can recognize interleukin 28B (IL28B) distinctly from interleukin 28A (IL28A). Although there are no more than 7 residues difference between IL28A and IL28B in a secreted form, the antibody according to the present invention can distinguish IL28B from IL28A, and enables specific detection and quantification of IL28B. IL28B is expected to be a marker for predicting beforehand whether HCV infection can be treated with pegylated interferon + ribavirin combination therapy or not. The present invention makes it possible to measure IL28B by simple, rapid and low-cost immunoassay, and thus can contribute to clinical practice of the therapy of HCV infections.

SEQUENCE LISTING

<110>  FUJIREBIO INC.

<120>  Anti-IL28B antibody and method for measurement of IL28B using the same

<130>  PF446-PCT

<160>  6

<170>  PatentIn version 3.1

<210>  1
<211>  734
<212>  DNA
<213>  Homo sapiens

<220>
<221>  CDS
<222>  (53)..(655)
<223>

<400>  1
tgggtgacag cctcagagtg tttcttctgc tgacaaagac cagagatcag ga atg aaa      58
                                                          Met Lys
                                                          1

cta gac atg act ggg gac tgc acg cca gtg ctg gtg ctg atg gcc gca      106
Leu Asp Met Thr Gly Asp Cys Thr Pro Val Leu Val Leu Met Ala Ala
        5                   10                  15

gtg ctg acc gtg act gga gca gtt cct gtc gcc agg ctc cac ggg gct      154
Val Leu Thr Val Thr Gly Ala Val Pro Val Ala Arg Leu His Gly Ala
    20                  25                  30

ctc ccg gat gca agg ggc tgc cac ata gcc cag ttc aag tcc ctg tct      202
Leu Pro Asp Ala Arg Gly Cys His Ile Ala Gln Phe Lys Ser Leu Ser
35                  40                  45                  50

cca cag gag ctg cag gcc ttt aag agg gcc aaa gat gcc tta gaa gag      250
Pro Gln Glu Leu Gln Ala Phe Lys Arg Ala Lys Asp Ala Leu Glu Glu
                55                  60                  65

tcg ctt ctg ctg aag gac tgc agg tgc cac tcc cgc ctc ttc ccc agg      298
Ser Leu Leu Leu Lys Asp Cys Arg Cys His Ser Arg Leu Phe Pro Arg
            70                  75                  80

acc tgg gac ctg agg cag ctg cag gtg agg gag cgc ccc atg gct ttg      346
Thr Trp Asp Leu Arg Gln Leu Gln Val Arg Glu Arg Pro Met Ala Leu
        85                  90                  95

gag gct gag ctg gcc ctg acg ctg aag gtt ctg gag gcc acc gct gac      394
Glu Ala Glu Leu Ala Leu Thr Leu Lys Val Leu Glu Ala Thr Ala Asp
    100                 105                 110

act gac cca gcc ctg gtg gac gtc ttg gac cag ccc ctt cac acc ctg      442
Thr Asp Pro Ala Leu Val Asp Val Leu Asp Gln Pro Leu His Thr Leu
115                 120                 125                 130

cac cat atc ctc tcc cag ttc cgg gcc tgt atc cag cct cag ccc acg      490
His His Ile Leu Ser Gln Phe Arg Ala Cys Ile Gln Pro Gln Pro Thr
                135                 140                 145

gca ggg ccc agg acc cgg ggc cgc ctc cac cat tgg ctg tac cgg ctc 538
Ala Gly Pro Arg Thr Arg Gly Arg Leu His His Trp Leu Tyr Arg Leu
            150                 155                 160

cag gag gcc cca aaa aag gag tcc cct ggc tgc ctc gag gcc tct gtc 586
Gln Glu Ala Pro Lys Lys Glu Ser Pro Gly Cys Leu Glu Ala Ser Val
        165                 170                 175

acc ttc aac ctc ttc cgc ctc ctc acg cga gac ctg aat tgt gtt gcc 634
Thr Phe Asn Leu Phe Arg Leu Leu Thr Arg Asp Leu Asn Cys Val Ala
    180                 185                 190

agt ggg gac ctg tgt gtc tga ccctcccacc agtcatgcaa cctgagattt 685
Ser Gly Asp Leu Cys Val
195                 200

tatttataaa ttagccactt gtcttaattt attgccaccc agtcgctat 734

<210> 2
<211> 200
<212> PRT
<213> Homo sapiens

<400> 2

Met Lys Leu Asp Met Thr Gly Asp Cys Thr Pro Val Leu Val Leu Met
1               5                   10                  15

Ala Ala Val Leu Thr Val Thr Gly Ala Val Pro Val Ala Arg Leu His
            20                  25                  30

Gly Ala Leu Pro Asp Ala Arg Gly Cys His Ile Ala Gln Phe Lys Ser
        35                  40                  45

Leu Ser Pro Gln Glu Leu Gln Ala Phe Lys Arg Ala Lys Asp Ala Leu
    50                  55                  60

Glu Glu Ser Leu Leu Leu Lys Asp Cys Arg Cys His Ser Arg Leu Phe
65                  70                  75                  80

Pro Arg Thr Trp Asp Leu Arg Gln Leu Gln Val Arg Glu Arg Pro Met
                85                  90                  95

Ala Leu Glu Ala Glu Leu Ala Leu Thr Leu Lys Val Leu Glu Ala Thr
            100                 105                 110

Ala Asp Thr Asp Pro Ala Leu Val Asp Val Leu Asp Gln Pro Leu His
        115                 120                 125

Thr Leu His His Ile Leu Ser Gln Phe Arg Ala Cys Ile Gln Pro Gln
    130                 135                 140

Pro Thr Ala Gly Pro Arg Thr Arg Gly Arg Leu His His Trp Leu Tyr

145                 150                 155                 160

Arg Leu Gln Glu Ala Pro Lys Lys Glu Ser Pro Gly Cys Leu Glu Ala
                165                 170                 175

Ser Val Thr Phe Asn Leu Phe Arg Leu Leu Thr Arg Asp Leu Asn Cys
            180                 185                 190

Val Ala Ser Gly Asp Leu Cys Val
        195                 200

<210>  3
<211>  603
<212>  DNA
<213>  Homo sapiens

<220>
<221>  CDS
<222>  (1)..(603)
<223>

<400>  3
atg aaa cta gac atg acc ggg gac tgc atg cca gtg ctg gtg ctg atg       48
Met Lys Leu Asp Met Thr Gly Asp Cys Met Pro Val Leu Val Leu Met
1               5                   10                  15

gcc gca gtg ctg acc gtg act gga gca gtt cct gtc gcc agg ctc cgc       96
Ala Ala Val Leu Thr Val Thr Gly Ala Val Pro Val Ala Arg Leu Arg
            20                  25                  30

ggg gct ctc ccg gat gca agg ggc tgc cac ata gcc cag ttc aag tcc      144
Gly Ala Leu Pro Asp Ala Arg Gly Cys His Ile Ala Gln Phe Lys Ser
        35                  40                  45

ctg tct cca cag gag ctg cag gcc ttt aag agg gcc aaa gat gcc tta      192
Leu Ser Pro Gln Glu Leu Gln Ala Phe Lys Arg Ala Lys Asp Ala Leu
    50                  55                  60

gaa gag tcg ctt ctg ctg aag gac tgc aag tgc cgc tcc cgc ctc ttc      240
Glu Glu Ser Leu Leu Leu Lys Asp Cys Lys Cys Arg Ser Arg Leu Phe
65                  70                  75                  80

ccc agg acc tgg gac ctg agg cag ctg cag gtg agg gag cgc ccc gtg      288
Pro Arg Thr Trp Asp Leu Arg Gln Leu Gln Val Arg Glu Arg Pro Val
                85                  90                  95

gct ttg gag gct gag ctg gcc ctg acg ctg aag gtt ctg gag gcc acc      336
Ala Leu Glu Ala Glu Leu Ala Leu Thr Leu Lys Val Leu Glu Ala Thr
            100                 105                 110

gct gac act gac cca gcc ctg ggg gat gtc ttg gac cag ccc ctt cac      384
Ala Asp Thr Asp Pro Ala Leu Gly Asp Val Leu Asp Gln Pro Leu His
        115                 120                 125

acc ctg cac cat atc ctc tcc cag ctc cgg gcc tgt atc cag cct cag      432
Thr Leu His His Ile Leu Ser Gln Leu Arg Ala Cys Ile Gln Pro Gln
    130                 135                 140

ccc acg gca ggg ccc agg acc cgg ggc cgc ctc cac cat tgg ctg cac      480

Pro Thr Ala Gly Pro Arg Thr Arg Gly Arg Leu His His Trp Leu His
145                 150                 155                 160

cgg ctc cag gag gcc cca aaa aag gag tcc cct ggc tgc ctc gag gcc      528
Arg Leu Gln Glu Ala Pro Lys Lys Glu Ser Pro Gly Cys Leu Glu Ala
            165                 170                 175

tct gtc acc ttc aac ctc ttc cgc ctc ctc acg cga gac ctg aat tgt      576
Ser Val Thr Phe Asn Leu Phe Arg Leu Leu Thr Arg Asp Leu Asn Cys
        180                 185                 190

gtt gcc agc ggg gac ctg tgt gtc tga                                  603
Val Ala Ser Gly Asp Leu Cys Val
    195                 200

<210>  4
<211>  200
<212>  PRT
<213>  Homo sapiens

<400>  4

Met Lys Leu Asp Met Thr Gly Asp Cys Met Pro Val Leu Val Leu Met
1               5                   10                  15

Ala Ala Val Leu Thr Val Thr Gly Ala Val Pro Val Ala Arg Leu Arg
            20                  25                  30

Gly Ala Leu Pro Asp Ala Arg Gly Cys His Ile Ala Gln Phe Lys Ser
        35                  40                  45

Leu Ser Pro Gln Glu Leu Gln Ala Phe Lys Arg Ala Lys Asp Ala Leu
    50                  55                  60

Glu Glu Ser Leu Leu Leu Lys Asp Cys Lys Cys Arg Ser Arg Leu Phe
65                  70                  75                  80

Pro Arg Thr Trp Asp Leu Arg Gln Leu Gln Val Arg Glu Arg Pro Val
                85                  90                  95

Ala Leu Glu Ala Glu Leu Ala Leu Thr Leu Lys Val Leu Glu Ala Thr
            100                 105                 110

Ala Asp Thr Asp Pro Ala Leu Gly Asp Val Leu Asp Gln Pro Leu His
        115                 120                 125

Thr Leu His His Ile Leu Ser Gln Leu Arg Ala Cys Ile Gln Pro Gln
    130                 135                 140

Pro Thr Ala Gly Pro Arg Thr Arg Gly Arg Leu His His Trp Leu His
145                 150                 155                 160

Arg Leu Gln Glu Ala Pro Lys Lys Glu Ser Pro Gly Cys Leu Glu Ala
                165                 170                 175

Ser Val Thr Phe Asn Leu Phe Arg Leu Leu Thr Arg Asp Leu Asn Cys
            180                 185                 190

Val Ala Ser Gly Asp Leu Cys Val
        195                 200

<210>  5
<211>  856
<212>  DNA
<213>  Homo sapiens

<220>
<221>  CDS
<222>  (98)..(700)
<223>

<400>  5
aattaccttt tcactttaca cacatcatct tggattgccc attttgcgtg gctaaaaagc      60

agagccatgc cgctggggaa gcagttgcga tttagcc atg gct gca gct tgg acc      115
                                         Met Ala Ala Ala Trp Thr
                                         1               5

gtg gtg ctg gtg act ttg gtg cta ggc ttg gcc gtg gca ggc cct gtc      163
Val Val Leu Val Thr Leu Val Leu Gly Leu Ala Val Ala Gly Pro Val
            10                  15                  20

ccc act tcc aag ccc acc aca act ggg aag ggc tgc cac att ggc agg      211
Pro Thr Ser Lys Pro Thr Thr Thr Gly Lys Gly Cys His Ile Gly Arg
        25                  30                  35

ttc aaa tct ctg tca cca cag gag cta gcg agc ttc aag aag gcc agg      259
Phe Lys Ser Leu Ser Pro Gln Glu Leu Ala Ser Phe Lys Lys Ala Arg
    40                  45                  50

gac gcc ttg gaa gag tca ctc aag ctg aaa aac tgg agt tgc agc tct      307
Asp Ala Leu Glu Glu Ser Leu Lys Leu Lys Asn Trp Ser Cys Ser Ser
55                  60                  65                  70

cct gtc ttc ccc ggg aat tgg gac ctg agg ctt ctc cag gtg agg gag      355
Pro Val Phe Pro Gly Asn Trp Asp Leu Arg Leu Leu Gln Val Arg Glu
                75                  80                  85

cgc cct gtg gcc ttg gag gct gag ctg gcc ctg acg ctg aag gtc ctg      403
Arg Pro Val Ala Leu Glu Ala Glu Leu Ala Leu Thr Leu Lys Val Leu
            90                  95                  100

gag gcc gct gct ggc cca gcc ctg gag gac gtc cta gac cag ccc ctt      451
Glu Ala Ala Ala Gly Pro Ala Leu Glu Asp Val Leu Asp Gln Pro Leu
        105                 110                 115

cac acc ctg cac cac atc ctc tcc cag ctc cag gcc tgt atc cag cct      499
His Thr Leu His His Ile Leu Ser Gln Leu Gln Ala Cys Ile Gln Pro
    120                 125                 130

cag ccc aca gca ggg ccc agg ccc cgg ggc cgc ctc cac cac tgg ctg      547
Gln Pro Thr Ala Gly Pro Arg Pro Arg Gly Arg Leu His His Trp Leu

135                 140                 145                 150

cac cgg ctc cag gag gcc ccc aaa aag gag tcc gct ggc tgc ctg gag      595
His Arg Leu Gln Glu Ala Pro Lys Lys Glu Ser Ala Gly Cys Leu Glu
                155                 160                 165

gca tct gtc acc ttc aac ctc ttc cgc ctc ctc acg cga gac ctc aaa      643
Ala Ser Val Thr Phe Asn Leu Phe Arg Leu Leu Thr Arg Asp Leu Lys
            170                 175                 180

tat gtg gcc gat ggg aac ctg tgt ctg aga acg tca acc cac cct gag      691
Tyr Val Ala Asp Gly Asn Leu Cys Leu Arg Thr Ser Thr His Pro Glu
        185                 190                 195

tcc acc tga caccccacac cttatttatg cgctgagccc tactccttcc              740
Ser Thr
    200

ttaatttatt tcctctcacc ctttatttat gaagctgcag ccctgactga gacatagggc    800

tgagtttatt gttttacttt tatacattat gcacaaataa acaacaagga attgga        856


<210>  6
<211>  200
<212>  PRT
<213>  Homo sapiens

<400>  6

Met Ala Ala Ala Trp Thr Val Val Leu Val Thr Leu Val Leu Gly Leu
1               5                   10                  15


Ala Val Ala Gly Pro Val Pro Thr Ser Lys Pro Thr Thr Thr Gly Lys
            20                  25                  30


Gly Cys His Ile Gly Arg Phe Lys Ser Leu Ser Pro Gln Glu Leu Ala
        35                  40                  45


Ser Phe Lys Lys Ala Arg Asp Ala Leu Glu Glu Ser Leu Lys Leu Lys
    50                  55                  60


Asn Trp Ser Cys Ser Ser Pro Val Phe Pro Gly Asn Trp Asp Leu Arg
65                  70                  75                  80


Leu Leu Gln Val Arg Glu Arg Pro Val Ala Leu Glu Ala Glu Leu Ala
                85                  90                  95


Leu Thr Leu Lys Val Leu Glu Ala Ala Ala Gly Pro Ala Leu Glu Asp
            100                 105                 110


Val Leu Asp Gln Pro Leu His Thr Leu His His Ile Leu Ser Gln Leu
        115                 120                 125


Gln Ala Cys Ile Gln Pro Gln Pro Thr Ala Gly Pro Arg Pro Arg Gly

130                 135                 140

Arg Leu His His Trp Leu His Arg Leu Gln Glu Ala Pro Lys Lys Glu
145                 150                 155                 160

Ser Ala Gly Cys Leu Glu Ala Ser Val Thr Phe Asn Leu Phe Arg Leu
                165                 170                 175

Leu Thr Arg Asp Leu Lys Tyr Val Ala Asp Gly Asn Leu Cys Leu Arg
            180                 185                 190

Thr Ser Thr His Pro Glu Ser Thr
        195                 200

## Description

### TECHNICAL FIELD

The present invention relates to an antibody which specifically binds to interleukin 28B, and a method for measurement of IL28B using the antibody.

### BACKGROUND ART

It is said that there are about 2,000,000 people infected with hepatitis C virus (HCV) in Japan. Hence, HCV infection is the most common infectious disease in Japan. Once people are infected with HCV, 60 to 80% of the infected people develop a chronic hepatitis. Chronic patients seldom recover spontaneously, and many of the chronic cases develop liver cirrhosis or liver cancer. Today in Japan, about 25,000 people die annually from liver cancer. Although HCV infections have been able to be completely cured by pegylated interferon + ribavirin combination therapy, no more than about 50% of HCV cases of genotype 1 with a high viral load, which are the most common in Japan, can be cured completely by the combination therapy, and about 20% of the cases do not respond to the combination therapy at all. The pegylated interferon + ribavirin combination therapy produces severe adverse effects with a high frequency, and in addition, is very expensive. Therefore, the combination therapy is a burden to patients, in particular, those for whom the combination therapy is not effective at all.

It is reported that the therapeutic effect of pegylated interferon + ribavirin combination therapy is strongly associated with interleukin 28B (IL28B) gene polymorphism and that the expression level of IL28B gene is significantly low in patients for whom the combination therapy is ineffective (Non-patent document 1). Hence, IL28B is expected to be a maker for predicting the therapeutic effect against HCV. However, neither of an IL28B specific antibody, nor a method and a kit for specific measurement of IL28B exist at present.

### PRIOR ART DOCUMENTS

### PATENT DOCUMENTS

Patent Document 1: JP 4350950 B

### NON-PATENT DOCUMENTS

Non-patent Document 1: Nature Genetics, vol. 41, No. 10, 2009, p.1105-1109

### SUMMARY OF THE INVENTION

### PROBLEMS TO BE SOLVED BY THE INVENTION

Accordingly, the present invention is to provide a novel means which enables immunoassay of IL28B.

### MEANS FOR SOLVING THE PROBLEMS

The present inventors tried to prepare a monoclonal antibody using recombinant IL28B as an immunogen and using recombinant IL28B and recombinant IL28A for antibody screening, and have succeeded in obtaining antibodies which bound to IL28B but did not substantially bind to IL28A. By epitope analyses of the obtained antibodies, they found that the epitope of the antibody was in the region of aa97-136 of the amino acid sequence of IL28B and that the antibody did not react with IL28B-G120V in which the 120th G (glycine) of IL28B was replaced by the 120th V (valine) of IL28A, and thus found that the antibody could specifically recognize the 120th G of IL28B, the amino acid different from the one of IL28A. They further found that the 114th D and the 124th D were also important for the binding of the antibody to IL28B. Furthermore, they established a method of sandwich immunoassay using the obtained antibody and another antibody which bound to IL28B at an epitope region different from that of the obtained antibody, and made it possible to provide an immunoassay kit, thereby completing the present invention.

That is, the present invention provides an antibody or antigen-binding fragment thereof, which binds to interleukin 28B by antigen-antibody reaction but does not substantially bind to interleukin 28A. The present invention also provides a method for measurement of interleukin 28B, comprising measuring interleukin 28B in a sample by immunoassay using the above-described antibody or antigen-binding fragment thereof according to the present invention. The present invention further provides a kit for immunoassay of interleukin 28B, comprising the above-described antibody or antigen-binding fragment thereof according to the present invention. The present invention still further provides a method for predicting the therapeutic effect of pegylated interferon + ribavirin combination therapy on HCV infection, said method comprising measuring interleukin 28B in a sample separated from an HCV-infected patient by the above-described method of the present invention and determining the expression level of interleukin 28B in said patient, wherein a low level expression of interleukin 28B indicates a high possibility that the combination therapy is not effective.

### EFFECTS OF THE INVENTION

By the present invention, an antibody which can recognize IL28B distinctly from IL28A was first provided. Although there are no more than 7 residues difference between IL28A and IL28B in a secreted form, the antibody according to the present invention can distinguish IL28B from IL28A, and enables specific detection and quantification of IL28B. It is known that the therapeutic effect of the pegylated interferon + ribavirin combination therapy on HCV infection is strongly associated with the IL28B gene polymorphism and that the expression level of IL28B gene is significantly low in patients for whom the combination therapy is not effective. Hence, IL28B is expected to be a marker for predicting beforehand whether HCV infection can be treated with the combination therapy or not. However, genetic polymorphism analysis is complicated, time-consuming, and in addition, expensive. The present invention makes it possible to measure IL28B by simple, rapid and low-cost immunoassay, and thus can contribute to clinical practice of the therapy of HCV infections.

### BRIEF DESCRIPTION OF THE DRAWINGS

Fig. 1 shows the alignment of the amino acid sequences of IL28A and IL28B.
Fig. 2 shows the results obtained by examining the reactivity of two monoclonal antibodies IL28-308 and IL28-402 produced in the Examples with IL28B, IL28A and IL29 by solid phase ELISA.
Fig.3 shows the results of measurement of IL28B and IL28A by sandwich ELISA established in the Examples.

### MODE FOR CARRYING OUT THE INVENTION

As described above, the antibody of the present invention binds to interleukin 28B (IL28B) by antigen-antibody reaction but does not substantially bind to interleukin 28A (IL28A). Such a binding property may be hereinafter described as "specifically bind to IL28B", and such an antibody may be referred to as "anti-IL28B antibody".

The term "does not substantially bind" herein means that the antibody does not bind to IL28A at a detectable level (i.e. its binding to IL28A is at the background level or below), or even if it binds to IL28A at a detectable level, the binding is very weak and apparently weaker than its binding to IL28B so that those skilled in the art would conclude that binding of the antibody to IL28B does not occur. Preferably, the anti-IL28B antibody of the present invention is an antibody that binds only to IL28B and does not bind to IL28A at a detectable level. For example, in the Examples below, the binding property of the produced antibodies to IL28A was checked by ELISA, and it can be concluded that binding at a detectable level does not occur if the level of binding detected by such a method is about the background level or below. In addition, the anti-IL28B antibody of the present invention does not substantially bind to IL29, preferably does not bind to it at a detectable level.

IL28A, IL28B and IL29 *per se* are known proteins and registered under accession numbers AY129148, AY129149 and AY129150 in GenBank, respectively. The registered sequences are shown in SEQ ID NOS: 1 to 6 in SEQUENCE LISTING. In the IL28B amino acid sequence shown in SEQ ID NO: 4, the region of the 1 st to the 25th amino acids (aa1-25) is a signal peptide, and the region of aa26-200 is secreted as a mature protein.

**[Table 1]**

| | SEQUENCE LISTING | GenBank accession No. |
|---|---|---|
| IL28A | SEQ ID NO: 1 (cDNA) | AY129148 |
| | SEQ ID NO: 2 (protein) | |
| IL28B | SEQ ID NO: 3 (cDNA) | AY129149 |
| | SEQ ID NO: 4 (protein) | |
| IL29 | SEQ ID NO: 5 (cDNA) | AY129150 |
| | SEQ ID NO: 6 (protein) | |

In the present invention, the position of a given amino acid residue is represented based on the amino acid sequence of IL28B with the full length of 200 residues including a signal peptide. For example, "the 120th amino acid" of IL28B refers to the 120th amino acid in the amino acid sequence shown in SEQ ID NO: 4. The residue is located at the 95th position from the N-terminal of IL28B in the mature form without a signal peptide.

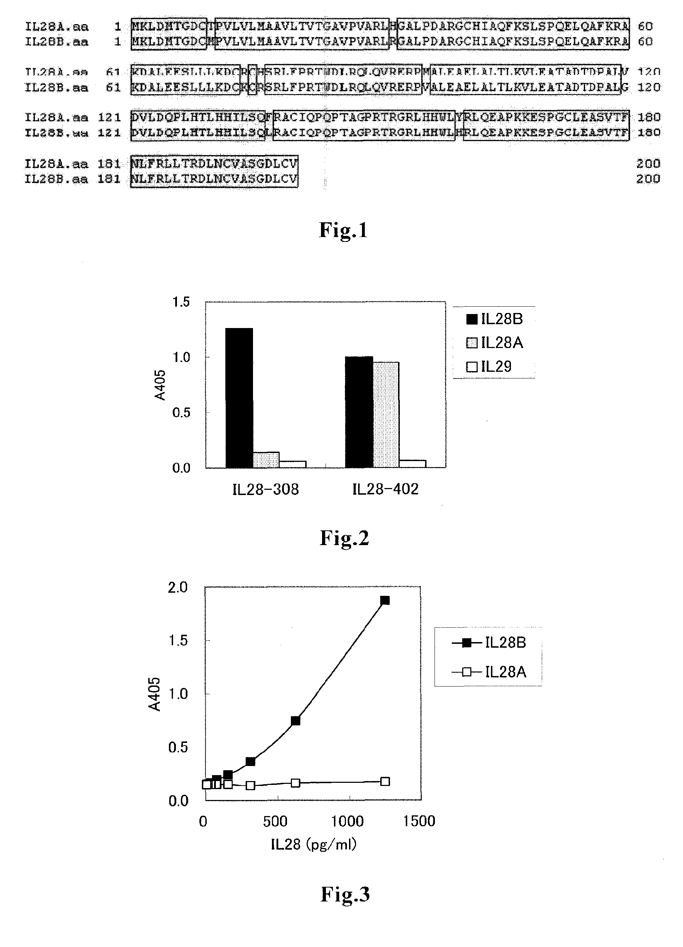

IL28B is a protein having a high homology to IL28A and IL29. Particularly, IL28B and IL28A have a very high homology to each other; the amino acid difference between them is 8 residues difference in the full length of 200 residues, and 7 residues difference in the secretory protein region (Fig. 1). The antibody of the present invention can specifically bind to IL28B distinctly from IL28A, which has such a very high homology to IL28B.

The anti-IL28B antibody preferably binds to an epitope located in the region of aa97-136 of IL28B. The expression "an epitope located in the region of aa97-136" encompasses an epitope consisting of the entire aa97-136 region and an epitope consisting of a partial region of the aa97-136 region. The anti-IL28B antibody produced in the Examples below binds to a partial fragment consisting of aa97-136 of IL28B but does not bind to a partial fragment consisting of the same region of IL28A. In addition, it has been confirmed that an antibody that binds to both IL28B and IL28A recognizes an epitope located in a different region. Therefore, an antibody whose epitope is any region (the entire region or a partial region thereof) located in the region of aa97-136 is an antibody which can specifically bind to IL28B.

The anti-IL28B antibody may recognize a specific amino acid residue(s) of IL28B. The phrase "recognize an amino acid residue(s)" means that an antibody binds to the residue(s) mentioned *per se* or to a partial structure on the surface of an antigen molecule which structure is constituted by the residue(s) mentioned, and includes not only cases where the anti-IL28B antibody binds to the residue(s) mentioned which appear(s) on the surface of the IL28B molecule but also cases where the residue(s) mentioned *per se* is(are) located inside of the molecular structure but is(are) critical in keeping the partial structure (including surface modifications such as carbohydrate structure) on the surface of the IL28B molecule to which the anti-IL28B antibody binds.

For example, the anti-IL28B antibody recognizes the 120th amino acid of IL28B. The 120th amino acid is glycine in IL28B and valine in IL28A. The anti-IL28B antibody produced in the Examples below does not bind to a mutant IL28B in which the 120th amino acid of IL28B is replaced by valine, but binds to a mutant IL28A in which the 120th amino acid of IL28A is replaced by glycine. Therefore, this anti-IL28B antibody is an antibody which recognizes the 120th amino acid of IL28B.

The anti- IL28B antibody may also recognize the 114th and the 124th amino acids besides the 120th amino acid. Although the 114th and the 124th amino acids are aspartic acid in both IL28B and IL28A, the anti-IL28B antibody described in the Example below loses its binding ability to IL28B when these amino acid residues are substituted. Hence, these residues may also be important components of the epitope of the anti-IL28B antibody.

The antibody according to the present invention may be a polyclonal antibody or a monoclonal antibody. Considering use of the antibody in immunoassays etc., a monoclonal antibody is preferred because of its high reproducibility.

The anti-IL28B antibody according to the present invention may be prepared by, for example, the well-known hybridoma method using the secretory protein region (aa26-200 of SEQ ID NO: 4) of IL28B as an immunogen. Specifically, for example, mature IL28B without a signal peptide is prepared by the well-known genetic engineering technique, and using it as an immunogen, an animal (except human) is immunized to induce antibodies in the animal body. Antibody producing cells such as spleen cells or lymphocytes are harvested from the animal, and the cells are fused with immortalized cells such as myeloma cells to prepare hybridomas. Using IL28B and IL28A as antigens for screening, a hybridoma which binds to IL28B but does not bind to IL28A is selected from the prepared hybridomas, and the selected hybridoma is grown so that an anti-IL28B monoclonal antibody can be obtained from supernatant of the hybridoma culture.

An antigen-binding fragment can be prepared from the antibody according to the present invention. The term "antigen-binding fragment" means an antibody fragment which maintains the binding ability (antigen-antibody reactivity) to the corresponding antigen of the antibody, such as a Fab fragment or a F(ab')₂ fragment of immunoglobulin. Such an antigen-binding fragment can also be used in immunoassays, as is well-known, and is also useful similarly to the original antibody. As is well-known, Fab and F(ab')₂ fragments can be obtained by treating a monoclonal antibody with a proteolytic enzyme such as papain or pepsin. The antigen-binding fragment is not restricted to a Fab fragment and a F(ab')₂ fragment, and may be any fragment which maintains the binding ability to the corresponding antigen, or may be one prepared by genetic engineering technique. In addition, for example, an antibody obtained by expressing a single chain fragment of variable region (scFv) in *E*. *coli* by genetic engineering technique may also be used. A method for preparation of scFv is also well-known, and scFv can be prepared by extracting mRNA from a hybridoma produced in the above-described manner to prepare single chain cDNA; performing PCR with primers specific for immunoglobulin H chain and L chain to amplify immunoglobulin H chain gene and L chain gene; joining those genes with a linker; adding thereto suitable restriction enzyme sites; introducing the resulting product into a plasmid vector; transforming *E*. *coli* with the vector; and by collecting scFv from *E*. *coli.* Such scFv is also included in the scope of the present invention as an "antigen-binding fragment".

By an immunoassay using the antibody or antigen-binding fragment thereof according to the present invention, IL28B can be specifically detected and quantified distinctly from IL28A although there are no more than 7 residues difference between IL28A and IL28B in a secreted form. The present invention also provides a method for measurement of interleukin 28B, comprising measuring interleukin 28B in a sample by immunoassay using the antibody or antigen-binding fragment thereof. In the present invention, the term "measurement" includes detection, quantification and semi-quantification.

Immunoassays *per se* are well-known in the art, and any of immunoassays may be used in the measurement method according to the present invention. That is, when classifying on the basis of the reaction type, known immunoassays include sandwich methods, competition methods, agglutination methods, Western blotting and the like, and when classifying on the basis of the label employed, known immunoassays include enzyme immunoassays, radio immunoassays, fluorescence immunoassays and the like. Any of these are included in the term "immunoassay" used in the present invention and may be employed in the measurement method according to the present invention. Specific examples of the immunoassay that can be preferably employed include, but not limited to, immunoprecipitation, ELISA, immunostaining, immunochromatography and the like. Sandwich methods such as sandwich ELISA and immunochromatography are more preferred from the viewpoint of rapid and simple detection of IL28B in medical practice.

Reagents necessary for each type of immunoassays are also well-known. Except that the antibody or antigen-binding fragment thereof according to the present invention is used as an antibody(ies), the immunoassay can be carried out using an ordinary immunoassay kit. That is, the present invention also provides a measurement kit for carrying out the measurement method according to the present invention, which kit comprising the above-described antibody or antigen-binding fragment thereof according to the present invention. Reagents included in the kit besides the antibody or antigen-binding fragment thereof may be the same as known ordinary immunoassay kits. For example, the measurement kit of the present invention may comprise a buffer which can be used as a sample diluent, wash solution or the like, an instruction manual, and the like.

The sample is not restricted at all, and may be any sample as long as it may contain IL28B. The sample may be a biological sample separated from a living body, or may be a sample not originated from a living body (e.g. an IL28B sample prepared by genetic engineering technique in a laboratory). Examples of the biological sample include, but not limited to, blood samples (such as whole blood, plasma, serum), cell extracts, tissue samples and the like. The living body is not restricted, and is a mammal such as human, dog, cat, rabbit, mouse, hamster and the like.

These immunoassays *per se* are well-known in the art, and so it is not necessary to explain these immunoassays in the present specification. Briefly, in sandwich immunoassays, for example, an antibody which binds to IL28B is immobilized on a solid phase (immobilized antibody). The immobilized antibody is then reacted with a sample, and after washing the solid phase, the resultant is reacted with an antibody labeled with a label (labeled antibody) which binds to IL28B at a site different from the site recognized by the immobilized antibody. After washing the solid phase, the labeled antibody bound to the solid phase is measured. By using an anti-IL28B antibody which specifically binds to IL28B as at least one of the immobilized antibody and the labeled antibody, IL28B in the sample can be measured distinctly from IL28A. An anti-IL28B antibody may be used as one of the immobilized antibody and the labeled antibody, and an anti-IL28A/B antibody which can also bind to IL28A may be used as the other. Or, two types of anti-IL28B antibodies which bind to the IL28B molecule at different sites may be used as the immobilized antibody and the labeled antibody. The anti-IL28A/B antibody may be obtained by, for example, selecting a hybridoma which produces an antibody which binds to both IL28A and IL28B in a screening step of hybridomas in the above-described preparation process of the anti-IL28B antibody. The immobilized antibody and the labeled antibody each may be a polyclonal antibody or a monoclonal antibody, and both is preferably a monoclonal antibody from the viewpoint of attaining a high accuracy in the measurement. In the immunoassay, an antigen-binding fragment of an antibody can be used instead of an antibody.

Measurement of the labeled antibody can be carried out by measuring a signal from the labeling substance. A method for measuring the signal is appropriately selected depending on the type of the labeling substance. For example, in the case of enzyme labels, a substrate of the enzyme is added to the reaction system, and the amount of coloring or luminescence which occurs by the enzymatic reaction is measured with an absorptiometer or a luminometer. Quantification of IL28B in a test sample can be carried out as follows. Immunoassay of standard samples which contain IL28B in known concentrations different from one another is carried out using the antibody or antigen-binding fragment thereof according to the present invention. The correlation between the amount of the signal from the label and the concentration of IL28B in the standard sample is plotted to prepare a calibration curve. Thereafter, the same operation is carried out on a test sample containing an unknown amount of IL28B to measure the amount of the signal from the label, and the measured amount is applied to the calibration curve.

In the case where both of the immobilized antibody and the labeled antibody are monoclonal antibodies, whether the combination of monoclonal antibodies is desirable or not can be easily determined by actually performing the immunoassay. If desired, whether the antibodies recognize different epitopes or not may be determined by identifying the recognition sites of the antibodies. Identification of the antibody recognition site can be carried out by a method well-known in the art. Briefly, for example, IL28B protein, which is the corresponding antigen, is partially digested with a proteolytic enzyme such as trypsin. A solution of the partial digestion products is then passed through an affinity column to which the antibody whose recognition site should be determined is bound, thereby allowing the digestion products to be bound to the column. Thereafter, the bound digestion products are eluted and subjected to conventional mass spectrometry so that the antibody recognition site can be identified.

HCV infections can be completely cured by pegylated interferon + ribavirin combination therapy, but the complete cure rate is low for genotype 1 cases with high viral load, which are the most common in Japan, and the combination therapy is not effective at all for about 20% of those cases. Recently, it is reported that the expression level of IL28B gene is significantly low in patients for whom the combination therapy is ineffective (Non-patent document 1), and thus IL28B is thought to be usable as a marker for prediction of the efficacy of HCV treatment. That is, IL28B may be measured by subjecting a sample separated from an HCV-infected patient to the method of the present invention to determine the IL28B expression level in the patient, and based on the measured expression level, whether an HCV-infected patient can be treated with pegylated interferon + ribavirin combination therapy can be predicted beforehand. A low level expression of IL28B indicates a high possibility that the combination therapy is not effective.

For example, the efficacy of the combination therapy for an HCV-infected patient can be predicted by determining the expression level of IL28B in known patients for whom the combination therapy has been effective to define "effective reference value", and comparing the measured value in the HCV-infected patient to the effective reference value. When there is a significant difference from the effective reference value (i.e. the measured value is significantly lower than the effective reference value), it is predicted that the combination therapy mentioned above will not be effective for the patient, and hence application of the above-mentioned combination therapy, which is expensive and also produces many adverse effects, can be avoided beforehand. When there is no significant difference from the effective reference value (i.e. the measure value is not significantly lower than the effective reference value), it is predicted that the patient is highly likely to be cured completely with the above-mentioned combination therapy, which will help to decide to positively apply the combination therapy. In addition to the effective reference value, the average expression level of IL28B in known patients for whom the combination therapy has not been effective (ineffective reference value) may also be determined, and the measured value may be further compared to the ineffective reference value.

### EXAMPLES

The present invention will now be described more concretely by way of an example thereof. However, the present invention is not restricted to the example below.

### 1. Preparation of Anti-Human IL28B Monoclonal Antibody

A recombinant human IL28B (rIL28B) was used as an immunogen. According to the sequence information of human IL28B (SEQ ID NO: 3), oligo DNAs were combined to obtain a DNA fragment, and cDNA of matured IL28B (a region encoding aa26 to aa200 of SEQ ID NO: 4) without a signal peptide was artificially synthesized by PCR from the obtained DNA fragment. After purification, cDNA was incorporated into a known expression vector and introduced into *E*. *coli,* and the expressed rIL28B was obtained by recovery and purification using a column. Mice were immunized with the obtained rIL28B (intraperitoneal administration, administered in an amount of about 20 µg/mouse, immunized 3 to 4 times), and a conventional hybridoma method was carried out to prepare hybridomas producing an antibody against the immunogen.

Antibody screening was carried out by ELISA and Western blotting (WB). In the screening, recombinant human IL28B and recombinant human IL28A (both of which were the matured form without a signal peptide, the regions of aa26 to aa200 of SEQ ID NO: 4 and SEQ ID NO: 2, respectively) prepared by a conventional method using baculovirus-insect cell expression system were used as antigens. Concrete procedure of antigen-immobilized ELISA is as follows.

Recombinant IL28B or recombinant IL28A was dissolved in PBS to a concentration of 0.5 µg/ml, and the resulting solution was added to a 96-well assay plate in an amount of 50 µl/well, followed by coating reaction at 37°C for 1 hour. After washing with PBST, a supernatant of hybridoma culture was added thereto in an amount of 50 µl/well, and the reaction was allowed to proceed at 37°C for 1 hour. After washing with PBST, POD-labeled anti-mouse IgG antibody was added thereto in an amount of 50 µl/well, and the reaction was allowed to proceed at 37°C for 1hour. After washing with PBST, coloring was carried out using ABTS substrate system, and the absorbance at A405 nm was measured with a microplate reader.

Antibodies that reacted with recombinant human IL28B but did not react with recombinant human IL28A were screened to obtain a plurality of monoclonal antibody lines specific to IL28B. A plurality of antibodies that bound to both IL28B and IL28A were also obtained. The obtained antibodies were checked for the reactivity by WB. A part of the results is shown in Table 2 below.

**[Table 2]**

| mAb | Specificity | Reactivity checked by WB | |
|---|---|---|---|
| | | IL28B | IL28A |
| IL28-202 | A/B | + | + |
| IL28-302 | B | + | - |
| IL28-307 | B | + | - |
| IL28-308 | B | + | - |
| IL28-310 | A/B | + | + |
| IL28-314 | B | + | - |
| IL28-316 | A/B | + | + |
| IL28-402 | A/B | + | + |

### 2. Cross-Reactivity Test of IL28 mAbs IL28-308 and IL28-402

The cross-reactivity of the antibodies was tested using an assay plate to which recombinant IL28B, IL28A, and IL29 were bound. Recombinant IL29 was purchased from R&D systems (#1598-IL-025).

Recombinant IL28B, recombinant IL28A, or recombinant IL29 was dissolved in PBS to a concentration of 0.5 µg/ml, and the resulting solution was added to a 96-well assay plate in an amount of 100 µl/well, followed by coating reaction at 37°C for 1 hour. After washing with PBST, 1% skim milk-PBS was added thereto in an amount of 250 µl/well, and masking was carried out at 37°C for 1 hour. The antibody was diluted to 200 ng/ml with 1% BSA-PBS and added thereto in an amount of 100 µl/well, and the reaction was allowed to proceed at 37°C for 1 hour. After washing with PBST, POD-labeled anti-mouse IgG antibody was added thereto in an amount of 100 µl/well, and the reaction was allowed to proceed at 37°C for 1hour. After washing with PBST, coloring was carried out using ABTS substrate system, and the absorbance at A405 nm was measured with a microplate reader.

The results are shown in Fig. 2. IL28-308 reacted with IL28B, but did not react with IL28A nor with IL29 (IL28B specific antibody). IL28-402 reacted with IL28B and also with IL28A, but did not react with IL29 (IL28 specific antibody).

### 3. Epitope Analysis of IL28 mAbs IL28-308 and IL28-402

Recombinant deletion mutants and point mutants of IL28B and IL28A were prepared, and the epitopes of the antibodies IL28-308 and IL28-402 obtained above were analyzed by WB. As for the point mutants, targeting the 7 amino acids in which IL28A and IL28B were different from each other, one amino acid residue of IL28B was replaced by the IL28A-type residue, or one amino acid residue of IL28A was replaced by the IL28B-type residue, thereby preparing point mutants. The results of WB are shown in Table 3 below.

**[Table 3]**

| | Reactivity Checked by WB | |
|---|---|---|
| Antigen | Monoclonal Antibody | |
| | IL28-308 | IL28-402 |
| IL28B wt | + | + |
| IL28A wt | - | + |
| IL28B aa97-136 | + | - |
| IL28A aa97-136 | - | - |
| IL28B R32H | + | + |
| IL28B K74R | + | + |
| IL28B R76H | + | + |
| IL28B V96M | + | + |
| IL28B G120V | - | + |
| IL28B L137F | + | + |
| IL28B H160Y | + | + |
| IL28A V120G | + | + |

IL28-308 reacted with IL28B but did not react with IL28A. This antibody reacted with the aa97-136 region of IL28B but did not react with the aa97-136 region of IL28A. In the aa97-136 region of IL28B, just one amino acid residue, i.e. the 120th glycine, is different from the residue of IL28A; in IL28A, the residue is valine. We examined the reactivity of the antibody with the point mutants with a substitution at this residue to find that IL28-308 did not react with IL28B G120V but reacted with IL28A V120G. In IL29, the amino acid corresponding to the 120th residue of IL28B is glutamine. Thus the 120th glycine is an amino acid specific to IL28B.

In addition to the 120th glycine, there are 6 positions where IL28B has different amino acids from IL28A; that is, 32nd, 74th, 76th, 96th, 137th, and 160th positions. We examined the reactivity of IL28B point mutants (IL28B R32A, K74R, R76H, V96M, L137F and H160Y) in which residues were replaced by the IL28A-type residues at these positions to find that IL28-308 reacted with these substitution mutants.

These results revealed that IL28-308 had an epitope in the aa97-136 region of IL28B and recognized the 120th glycine.

On the other hand, it was revealed that IL28-402 was an antibody which reacted with IL28B and IL28A and which had its epitope in a region different from the epitope region of IL28-308 (in a region other than aa97-136).

### 4. Detailed Analysis on IL28 mAb IL28-308 Epitope

The 114th to the 125th amino acid residues of IL28B were replaced by alanine to prepare the IL28B alanine point mutants, and the antibody epitope was analyzed by WB further in detail. The results of WB are shown in Table 4 below.

**[Table 4]**

| | Reactivity Checked by WB |
|---|---|
| Antigen | Monoclonal Antibody |
| | IL28-308 |
| IL28B wt | + |
| IL28B D114A | - |
| IL28B T115A | + |
| IL28B D116A | + |
| IL28B P117A | + |
| IL28B L119A | + |
| IL28B G120V | - |
| IL28B D121A | + |
| IL28B V122A | + |
| IL28B L123A | + |
| IL28B D124A | - |
| IL28B Q125A | + |

IL28-308 did not react with the substitution mutant G120V and also not with D114A nor D124A, which revealed that not only the 120th G but also the 114th D and the 124th D were important for the binding of the antibody IL28-308 to IL28B.

### 5. Epitope Analysis of IL28 mAb IL28-402

Recombinant deletion mutants of IL28B were prepared to analyze the antibody epitope by WB. The results are shown in Table 5.

**[Table 5]**

| | Reactivity Checked by WB |
|---|---|
| Antigen | Monoclonal Antibody |
| | IL28-402 |
| IL28B wt | + |
| IL28B aa26-200 | + |
| IL28B aa60-200 | - |
| IL28B aa26-60 | + |

IL28-402 did not react with the aa60-200 region but reacted with the aa26-60 region, which revealed that the antibody IL28-402 was an antibody whose epitope was in the aa26-60 region of IL28B.

### 6. Establishment of IL28B Measurement System by Sandwich ELISA

Among the obtained antibodies, those which had been purified were labeled with biotin, and combinations with various IL28 mAbs for sandwich method were examined. As a labeled antibody, three lines, i.e. IL28-308 (anti-B), IL28-314 (anti-B) and IL28-402 (anti-A/B) were used. As an immobilized antibody, IL28-302 (anti-B), 307 (anti-B) and 316 (anti-A/B) were used in addition to the above-described antibodies. As a result, it was confirmed that sandwich method was possible in cases of the combination of labeled 402 with 302, 307, 308, 314, or 316 and the combination of labeled 308 or labeled 314 with 402.

Next, measurement of rIL28B and rIL28A was carried out by sandwich ELISA with immobilized IL28-402 and labeled IL28-308. A solution of 10 µg/ml IL28-402 in 0.1M citrate buffer (pH 3.5) was prepared and added to a 96-well assay plate in an amount of 100 µl/well, and coating was carried out at 37°C for 1 hour. After masking with 1% BSA-PBS and washing with PBST, antigen solutions prepared by serial dilution of rIL28B or rIL28A were added thereto in an amount of 100 µl/well, and the reaction was allowed to proceed at 37°C for 1 hour. After washing with PBST, biotinylated IL28-308 was added thereto in an amount of 100 µl/well, and the reaction was allowed to proceed at 37°C for 1 hour. After washing with PBST, streptavidin-alkaline phosphatase was added thereto in an amount of 100 µl/well, and the reaction was allowed to proceed at 37°C for 1 hour. After washing with PBST, coloring was carried out using p-NPP substrate system, and the absorbance at A405 nm was measured with a microplate reader.

Results are shown in Fig. 3. By sandwich ELISA with immobilized IL28-402 and labeled IL28-308, IL28B was measured in a concentration-dependent manner, and cross-reaction with IL28A was not substantially observed. Thus it was confirmed that the IL28B-specific measurement method could be established.

## Claims

1. An antibody or antigen-binding fragment thereof, which binds to interleukin 28B by antigen-antibody reaction but does not substantially bind to interleukin 28A.

2. The antibody or antigen-binding fragment thereof according to claim 1, which does not substantially bind to interleukin 29.

3. The antibody or antigen-binding fragment thereof according to claim 1 or 2, which binds to an epitope located in the region of the 97th to the 136th amino acids of interleukin 28B.

4. The antibody or antigen-binding fragment thereof according to claim 3, which recognizes the 120th amino acid of interleukin 28B.

5. The antibody or antigen-binding fragment thereof according to claim 4, which further recognizes the 114th and the 124th amino acids of interleukin 28B.

6. The antibody or antigen-binding fragment thereof according to claim 4, which binds to an interleukin 28A mutant with the 120th amino acid replaced by glycine, but does not bind to an interleukin 28B mutant with the 120th amino acid replaced by valine.

7. The antibody or antigen-binding fragment thereof according to any one of claims 1 to 6, wherein said antibody is a monoclonal antibody.

8. A method for measurement of interleukin 28B, comprising measuring interleukin 28B in a sample by immunoassay using the antibody or antigen-binding fragment thereof according to any one of claims 1 to 7.

9. The method according to claim 8, wherein the immunoassay is carried out by sandwich method using an immobilized antibody immobilized on a solid phase and a labeled antibody labeled with a label, and wherein the antibody or antigen-binding fragment thereof according to any one of claims 1 to 7 is used as at least one of the immobilized antibody and the labeled antibody.

10. A kit for immunoassay of interleukin 28B, comprising the antibody or antigen-binding fragment thereof according to any one of claims 1 to 7.

11. The kit according to claim 10, comprising said antibody or antigen-binding fragment thereof as at least one of an immobilized antibody immobilized on a solid phase and a labeled antibody labeled with a label.

12. A method for predicting the therapeutic effect of pegylated interferon + ribavirin combination therapy on HCV infection, said method comprising measuring interleukin 28B in a sample separated from an HCV-infected patient by the method according to claim 8 or 9 and determining the expression level of interleukin 28B in said patient, wherein a low level expression of interleukin 28B indicates a high possibility that the combination therapy is not effective.
